# EUROPEAN PATENT APPLICATION

(11) **EP 4 053 283 A1**
(43) Date of publication of application: **07.09.2022**
(21) Application number: 20882295.7
(22) Date of filing: 29.10.2020
(51) Int. Cl.: C12N 15/74, C12N 9/12, C12N 9/16, C12Q 1/02, A61K 35/74, A61P 35/00, G01N 21/76

(54) **TUMOR-TARGETING SALMONELLA GALLINARUM STRAIN AND USE THEREOF**

(30) Priority: 31.10.2019 KR 20190138224
(71) Applicant: Industry Foundation of Chonnam National University, Gwangju 61186 (KR)
(72) Inventor: CHOY, Hyon El, Gwangju 61503 (KR); JEONG, Jae Ho, Gwangju 61967 (KR); LIM, Dae Jin, Gwangju 61736 (KR); LIM, Hyung Ju, Gwangju 61508 (KR); KIM, Kwangsoo, Gwangju 61697 (KR)
(74) Representative: Plougmann Vingtoft a/s
(86) International application number: PCT/KR2020/014965
(87) International publication number: WO 2021/086085

(57) **Abstract**

The present invention relates to a tumor-targeting *Salmonella gallinarum* strain and the use thereof. The tumor-targeting *Salmonella gallinarum* strain has excellent tumor proliferation inhibitory activity and enables tumor-specific targeting, and thus can be utilized for treatment and imaging of tumors without any side effects.

## Description

### Technical Field

The present disclosure relates to a tumor-targeting *Salmonella gallinarum* strain and a use thereof.

### Background Art

Cancer is one of the most common causes of death, and recently, the incidence of cancer is on the rise due to environmental factors including environmental pollutants such as air pollution, and personal factors including high-fat intake due to westernization of diet, drinking alcohol, and smoking increase. Therefore, the development of anticancer materials for early prevention and treatment of cancer is becoming important. Compound-based anticancer drugs act on cancerous cells, but often exhibit non-specificity affecting the whole body, with the problem of adverse effects.

Since bacterial suppression of cancer has been reported, studies have been conducted into strains exhibiting anticancer effects. In the last 20 years, studied have been focused on the use of viable bacteria for cancer therapy, finding diverse variants of *Bifidobacterium, Clostridium, Lactococcus, Shigella, Vibrio, Listeria, Escherichia,* and *Salmonella* spp. in cancer animal models. However, the basic mechanism of cancer treatment using such strains is not fully understood yet.

Among them, *Salmonella* spp. have been studied for use as an anticancer target carrier and use for inhibiting cancer growth (patent document 1). However, the Salmonella strains studied previously exhibit low cancer cell targeting rates because of their distribution in various organs as well as cancerous cells. In addition, adverse effects are caused by the strains themselves. Particularly in patients or the elderly with weakened immune function, even the Salmonella strain of lowered pathogenicity may cause fatal sepsis. In addition, the conventional Salmonella strains have difficulty in achieving effective therapy and delivering anticancer substance into cells due to their low viability in cancer tissues.

With respect to *Salmonella gallinarum* strains, a vaccine composition and a feed composition for prevention of fowl typhoid are disclosed (patent document 2), but nowhere is the cancer treatment efficacy of the strains described.

Leading to the present disclosure, intensive and thorough research conducted by the present inventors resulted in the finding that an attenuated *Salmonella gallinarum* strain deficient in guanosine tetraphosphate (ppGpp) synthesis exhibits superiority in targeting and viability for cancer cells, but inferiority in targeting and viability for normal organs, compared to conventional Salmonella typhimurium strains and as such, can be effectively used for cancer therapy.

### [Related Art Document]

### [Patent Literature]

(Patent Literature 1) Korean Patent No. 10-2015573
(Patent Literature 2) Korean Patent No.10-2011-0126227 A

### Disclosure of Invention

### Technical Problem

An aspect of the present disclosure is to provide an attenuated *Salmonella gallinarum* strain lacking guanosine tretraphosphate (ppGpp) production capability.

Another aspect of the present disclosure is to provide a pharmaceutical composition comprising the *Salmonella gallinarum* strain for prevention or treatment of tumor.

A further aspect of the present disclosure is to provide a method for providing information about tumor analysis, the method comprising the steps of: administering the *Salmonella gallinarum* strain to a subject; detecting bioluminescence of the strain; and determining the presence of tumor in the subject if the bioluminescence is detected.

In the present disclosure, the subject may be animals including or except for humans.

### Solution to Problem

The present inventors have conducted research into the development of a Salmonella strain with targeting and viability excellent for cancer cells, but poor for normal organs, which leads to the present disclosure.

An aspect of the present disclosure provides an attenuated *Salmonella gallinarum* strain lacking guanosine tetraphosphate (ppGpp) production capability.

Being able to specifically target tumors, the attenuated *Salmonella gallinarum* lacking ppGpp production capability according to the present disclosure allows for effective therapy for tumors with minimal damages on normal organs except for tumorous tissues and thus can find advantageous applications in preventing, alleviating, or treating tumors.

As used herein, the term "attenuated" refers to modified to lower pathogenicity of a strain. Attenuation makes it possible to prevent the pathogenicity of the strain from causing cytotoxicity and other adverse effects in normal cells other than tumor cells. Attenuated strains can be constructed using various methods known in the art. By way of example, the attenuation is made by deletion or destruction of a virulence factor so that the strain can be viable in host cells. In this regard, *pab, proBC, nadA, pncB, pmi, rpsL, ompR, htrA, hemA, rfc, poxA, galU, aro, galE, cya, cyp, crp, cdt, pur, phoP, phoQ, ssa, guaA, guaB, clpP, clpX fliD, flgK, flgL, relA, spoA,* and *spoT* genes, and the genes, encoded by Salmonella Pathogenicity Islands, including ssaV, sseBCD, ssrAB, sopB, sseF, and sseG, can be deleted to achieve the attenuation.

According to an embodiment of the present disclosure, the strain is a variant of the *Salmonella gallinarum* SG4021 strain (microorganism accession number: KCTC13985BP) as a result of deleting guanosine tetraphosphate production capability therefrom.

The *Salmonella gallinarum* SG4021 strain is a clinical isolate from the livers of chickens with fowl typhoid, reared in a Korean chicken farm and was deposited with the Korean Research Institute of Bioscience and Biotechnology (microorganism accession number: KCTC13985BP) on October 8, 2019.

According to an embodiment of the present disclosure, the strain may be *Salmonella gallinarum* SG4023 (microorganism accession number: KCTC14338BP).

According to an embodiment of the present disclosure, the strain may lack a gene coding for ppGpp synthetase and may be attenuated as a result of deleting a gene coding for ppGpp synthetase.

The term "ppGpp", as used herein, refers to guanosine tetraphosphate and may be interchangeably used with guanosine 5'-diphosphate 3'-diphosphate or guanosine 3',5'-bispyrophosphate.

According to an embodiment of the present disclosure, the gene may include relA and spoT.

ppGpp, which is an intracellular signal transduction messenger, functions to induce the expression of genes encoded by Salmonella pathogenicity island (SPI) among the genes responsible for the toxicity of *Salmonella gallinarum* strains, and is encoded by *relA* and *spoT* genes. Therefore, *Salmonella gallinarum* strain lacking *relA* and *spoT* genes may become weaker in pathogenicity by one million fold or greater than the wild-type Salmonella, so that effective attenuation is possible.

The ppGpp-deficient variant according to the present disclosure may be produced by deleting both the relA and spoT genes.

The deletion of both *relA* and *spoT* genes may be made by modification of *relA* and *spoT* genes to bring about impairment into the transcription or translation of the genes or the activity of the gene products. Such genetic modifications may include inactivation of not only an ppGpp synthetase coding sequence, but also a promoter thereof. Specific inactivation of only a target gene on the genome of *Salmonella gallinarum* may be achieved by a mutation through substitution, insertion, deletion, or a combination thereof on the entire area or one partial area of the coding gene. For example, the deletion of a gene or the insertion of a heterogenous sequence into a gene may result in gene truncation, a nonsense mutation, a frameshift mutation, a missense mutation, and so on. Such specific gene inactivation can be carried out using a method usually used in the art. The deletion of a gene may be conducted using various mutagenesis methods known in the art. For example, the deletion of *relA* and *spoT* genes may be achieved by PCR mutagenesis and cassette mutagenesis.

According to an embodiment of the present disclosure, the strain may lack a *glmS* gene.

According to an embodiment of the present disclosure, the strain may be *Salmonella gallinarum* SG4031 (microorganism accession number: KCTC14339BP).

The *glmS* gene-deficient *Salmonella gallinarum* can be lysed in the animal system due to the lack of the peptidoglycan constituent D-glucosamine (GlcN) or N-acetyl-D-glucosamine (GlcNAc). Thus, the gene can be used as a selective determinant for the strain.

With respect to the method of deleting a *glmS* gene, the contents overlapping with the above description are omitted to avoid undue complexity of the specification.

According to an embodiment of the present disclosure, the strain may be a transformant anchoring a plasmid carrying a *glmS* gene operatively linked to a promoter. In detail, the strain may have a glmS-deleted mutation on the chromosome thereof.

According to an embodiment of the present disclosure, a balanced-lethal host-vector system may be constructed by transformation of a plasmid carrying a *glmS* gene for compensating for a *glmS* deleted mutation on the chromosome thereof. The attenuated *Salmonella gallinarum* strain into which a plasmid carrying a *glmS* gene has been successfully transformed can synthesize GlcN or GlcNAc and survive even a GlcN- or GlcNAc-insufficient environment. Thus, the attenuated strain can serve as a selection marker.

The term "operatively linked", as used herein, refers to functional linkage between a gene expression control sequence and a different nucleotide sequence. The gene expression control sequence may be at least one selected from the group consisting of a replication origin, a promoter, and a transcription terminator. The transcription terminator may be a polyadenylation sequence (pA) and examples of the replication origin include f1 replication origin, SV40 replication origin, pMB1 replication origin, adeno replication origin, AAV replication origin, and BBV replication origin, but are not limited thereto.

As used herein, the term "promoter" refers to a region of DNA, located upstream of a structural gene, to which an RNA polymerase binds to initiate transcription.

A promoter according to an embodiment of the present disclosure may be a polynucleotide fragment about 100 bp to about 2500 bp long, functioning as a transcriptional regulatory sequence for regulating the transcription initiation of a specific gene. For example, the promoter may be selected from the group consisting of tac promoter, *lac* promoter, *lacUV5* promoter, *lpp* promoter, *pLλ* promoter, *pRλ* promoter, *rac5* promoter, *amp* promoter, *recA* promoter, *SP6* promoter, trp promoter, *T7* promoter, *pBAD* promoter, *Tet* promoter, trc promoter, *pepT* promoter, *sulA* promoter, *pol 11*(*dinA*) promoter, *ruv* promoter, *uvrA* promoter, *uvrB* promoter, *uvrD* promoter, *umuDC* promoter, *lexA* promoter, *cea* promoter, *caa* promoter, *recN* promoter, *pagC* promoter, and a synthetic promoter, but with no limitations thereto.

A plasmid according to an embodiment of the present disclosure may be any one used in the art and may be, for example, selected from the group consisting of pcDNA seriese, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, but with no limitations thereto.

The plasmid of the present disclosure may include at least one selective marker. The marker is a nucleic acid sequence characterized by ability to be selected by a chemical method and may be accounted for by a gene that allows the discrimination of transformed cells from non-transformed cells. For example, the marker may be a gene resistant to a herbicide, such as glyphosate, glufosinate ammonium, or phosphinothricin; or a gene resistant to an antibiotic such as ampicillin, kanamycin, G418, bleomycin, hygromycin, or chloramphenicol, but with no limitations thereto.

The plasmid of the present disclosure may be constructed using a genetic recombination technique known in the art, and site-specific DNA cleavage and ligation may be carried out using enzymes generally known in the art.

The plasmid of the present disclosure may be transformed into the *Salmonella gallinarum* strain by a method that is typically used in the art to introduce a nucleic acid into cells. The transformation may be carried out a suitable standard technique known in the art. For example, the transformation method may be electroporation, calcium phosphate (CaPO₄) precipitation, calcium chloride (CaCl₂) precipitation, microinjection, and use of polyethylene glycol (PEG), DEAE-dextran, cationic liposome, and lithium acetate-DMSO, but with no limitations thereto.

According to an embodiment of the present disclosure, the plasmid may further include a luminescence gene.

As used herein, the term "luminescence gene" refers to a gene encoding a protein that allows for luminescence imaging in vivo or ex vivo (in vitro) and is intended to encompass bioluminescence genes (e.g., luciferase gene), chemiluminescence genes, and fluorescence genes of various origins. An imaging device for the luminescence phenomenon expressed by such genes is known in the art and may be appropriately selected by a person skilled in the art.

Targeting tumors, the *Salmonella gallinarum* strain transformed with a plasmid including a luminescence gene according to an embodiment of the present disclosure allows tumors to be visibly monitored and as such, can increase the accuracy and effect of tumor therapy. An imaging device that can be used in association with the luminescence gene of the present disclosure may be appropriately selected by a person skilled in the art.

According to an embodiment of the present disclosure, the luminescence gene may be *luxCDABE.*

Herein, "*luxCDABE*" is a DNA fragment including all a gene coding for luciferase (heterodimer of *luxA* and *luxB*) and genes (l*uxC, luxD,* and *luxE*) coding for tetradecanal, which is a luciferase substrate. The light-emitting compound luciferin is activated by ATP in cells and the active luciferin undergoes a luciferase-catalyzed reaction with oxygen with the concomitant emission of light through conversion from chemical energy to light energy.

Another aspect of the present disclosure provides a pharmaceutical composition comprising the *Salmonella gallinarum* strain for prevention or treatment of tumors.

The pharmaceutical composition of the present disclosure may include a pharmaceutically acceptable carrier. So long as it is typically used for preparing medications, any pharmaceutically acceptable carrier may be included in the pharmaceutical composition of the present disclosure. Examples of the pharmaceutically acceptable carrier include lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, and mineral oil, but are not limited thereto. In addition to the ingredients, the pharmaceutical composition of the present disclosure may further include a lubricant, a humectant, a sweetener, an emulsifier, a suspending agent, a preservative, and so on. For details of suitable pharmaceutically acceptable carriers and compositions, reference may be made to *Remington: the science and practice of pharmacy* 22nd edition (2013).

The pharmaceutical composition of the present disclosure may include various bases and/or additives necessary and suitable for formulations and can be formulated together with a non-ionic surfactant, a silicon polymer, a extender pigment, an aromatic, a preservative, a sterilizer, an oxidation stabilizer, an organic solvent, an ionic or non-ionic thickener, a softener, an antioxidant, a free radical scavenger, an opacifier, an emollient, silicone, α-hydroxy acid, a foaming agent, a moisturizer, a vitamin, an insect repellent, a flavorant, a preservative, a surfactant, an anti-inflammatory agent, a substance P antagonist, a filler, a polymer, a propellant, an alkalifying or acidifying agent, or a colorant within such an amount range as not to degrade the effect thereof.

A suitable dose of the pharmaceutical composition of the present disclosure may be prescribed depending on various factors including formulation methods, administration modes, the patient's age, body weight, sex, illness condition, and diet, the time of administration, the route of administration, excretion rates, and responsiveness. The pharmaceutical composition of the present disclosure may be administered at a dose of 0.001 to 1000 mg/kg to an adult. Meanwhile, the dosage unit may range from, for example, about 10⁴ to 10¹⁰ CFU, but with no limitations thereto. It may be administered once or in divided forms a day and a daily dosage may be 10⁴ to 10¹⁰ CFU, but with no limitations thereto.

The pharmaceutical composition of the present disclosure may be parenterally administered.

The pharmaceutical composition of the present disclosure may be parenterally administered and preferably intravenously, but with no limitations thereto.

For a formulation in a parenteral dosage form, for example, the pharmaceutical composition of the present disclosure may be mixed with a stabilizer or buffer in water to give a solution or suspension which is then prepared into ampule or vial dosage units. In addition, the composition may further include an auxiliary additive such as a stabilizer, a wettable powder or an emulsificant, a salt for adjustment of an osmotic pressure, and a buffer, and other therapeutically effective materials and may be formulated using a typical method.

According to an embodiment of the present disclosure, the tumor may be a solid tumor.

According to an embodiment of the present disclosure, the tumor may be adenocarcinoma.

According to an embodiment of the present disclosure, the tumor may be selected from the group consisting of colorectal cancer, pancreatic cancer, skin cancer, and breast cancer.

Adenocarcinoma is a type of cancerous tumor that occurs in cells of glands, including mucous membranes such as in stomach, intestine, bronchus, uterine, gall bladder, etc., glandular tissues or excretory ducts such as in prostate, thyroid gland, and pancreas.

According to previous studies on anticancer targeting therapy using bacteria, it is reported that immune responses and cytokine delivery in tumor cells can induce an anticancer immune effect without the adverse effect of systemic toxicity (Saltzman DA, Heise CP, Hasz DE, et al Attenuated Salmonella typhimurium containing interleukin-2 decreases MC-38 hepatic metastases: A novel anti-tumor agent Cancer Biother Radio 1996;11:145-53; Sorenson BS, Banton KL, Frykman NL, Leonard AS, Saltzman DA Attenuated salmonella typhimurium with IL-2 gene reduces pulmonary metastases in murine osteosarcoma Clin Orthop Relat R 2008;466:1285-91) and that the growth of cancer cells is inhibited by inducing the deposition of leucocytes and neutrophils around tumors (Loeffler M, Le'Negrate G, Krajewska M, Reed JC Attenuated Salmonella engineered to produce human cytokine LIGHT inhibit tumor growth Proceedings of the National Academy of Sciences of the United States of America 2007;104:12879-83; Loeffler M, Le'Negrate G, Krajewska M, Reed JC Salmonella typhimurium engineered to produce CCL21 inhibit tumor growth Cancer Immunol Immun 2009;58:769-75).

In the present disclosure, the targeting activity of *Salmonella gallinarum* for colorectal cancer, pancreatic cancer, skin cancer, and breast cancer cell lines was found, together with the confirmation of the excellent anticancer effect of ΔppGpp *Salmonella gallinarum* strain according to an embodiment of the present disclosure in a mouse model of CT26 tumor, which is a type of adenocarcinoma. In this context, thus, the *Salmonella gallinarum* strain according to an embodiment of the present disclosure can exhibit various inhibitory activities against adenocarcinoma by targeting various adenocarcinomas such as colorectal cancer, pancreatic cancer, skin cancer, and breast cancer, and inducing the expression of cytokines and/or the deposition of leukocytes.

A further aspect of the present disclosure provides a method for providing information about tumor analysis, the method comprising the steps of: administering the *Salmonella gallinarum* strain to a subject; detecting bioluminescence of the strain; and determining the presence of tumor in the subject if the bioluminescence is detected.

With respect to the method of injecting the *Salmonella gallinarum* strains to a subject and the method of detecting bioluminescence, the contents overlapping with the above description are omitted to avoid undue complexity of the specification.

The tumor cells targeted by the *Salmonella gallinarum* strain transformed with a luminescence gene according to an embodiment of the present disclosure can be detected due to the luminescence gene whereby information about the presence or absence of tumor in the subject and the position, size, and temporal change pattern of the tumor present in the subject can be acquired.

### Advantageous Effects of Invention

According to the tumor targeting Salmonella gallinarum strain and the use thereof, the strain has excellent inhibitory activity against tumor growth and can specifically target tumors, thus finding advantageous applications in tumor therapy and imaging without adverse effects.

### Brief Description of Drawings

FIG. 1 shows plots of survival rates of BALB/c mice according to doses of (A) the wild-type Salmonella gallinarum strain (SG4021) or (B) the ppGpp-deleted Salmonella gallinarum (SG4023) after intravenous injection.
FIG. 2 shows plots illustrating comparison of plasmid stability between the Salmonella strain (SG4033) and lacking glmS and ppGpp genes that was transformed with the GlmS+pLux plasmid and the Salmonella gallinarum strain (SG4032) lacking ppGpp that was transformed with the GlmS+pLux plasmid.
FIG. 3 shows images obtained through bioluminescence signal imaging device for results of targeting various tumors after intravenous injection of the Salmonella gallinarum strain (SG4033, ca. 1×10⁸ CFU) transformed with the GlmS+pLux plasmid.
FIG. 4 shows images of CT26 tumor tissues stained with DAPI (nucleus), alexa Fluor 488-conjugated Phalloidin (actin), and a Salmonella gallinarum-specific antibody three days after injection of the ppGpp-deleted Salmonella gallinarum strain (SG4023, ca. 1×10⁸ CFU).
FIG. 5 shows (A) images of bioluminescence signals expressed with time from the GlmS+pLux plasmid transformed into the ppGpp-deleted Salmonella gallinarum strain (SG4032) including the wild-type glmS and the ppGpp-deleted Salmonella gallinarum strain (SG4033) lacking glmS, which targeted CT26 tumor tissues after intravenous injection (ca. 1×108 CFU) and (B) a graph of fractions of Salmonella strains including the GlmS+pLux plasmid seven days after injection of the Salmonella strains.
FIG. 6 shows (A) a plot of tumor volumes with time in CT26 cell-implanted BALB/c mice to which ca. 1×10⁸ CFU of the ΔppGpp Salmonella gallinarum (SG4023) or ca. 1×10⁷ CFU of the Salmonella typhimurium strain (SMR2130) was intravenously injected, (B) images illustrating changes of the implant tumors, and (C) a plot of survival rates of the mice.
FIG. 7 shows plots of numbers of Salmonella strains with time in various organs of CT26 cell-implanted BALB/c mice to which (A) ca. 1×10⁸ CFU of the ΔppGpp Salmonella gallinarum strain (SG4022) and (B) ca. 1×10⁷ CFU of the ΔppGpp Salmonella typhimurium (SHJ2037) were intravenously injected.

### Best Mode for Carrying out the Invention

A better understanding of the present disclosure will be obtained from the following Examples which are set forth to illustrate the present disclosure and are not to be construed as limiting the present disclosure.

### EXAMPLE 1. Preparation of Attenuated Salmonella gallinarum Strain and Assay for Tumor Inhibition Activity

### 1-1. Salmonella strain growth

*Salmonella spp.* were grown at 37°C in LB medium containing 1% NaCl (Difco Laboratories) under vigorous aeration. A solid support medium was prepared with 1.5% granule agar (Difco Laboratories). Antibiotics were purchased from Sigma Chemical. If necessary, the antibiotics ampicillin, kanamycin, and chloramphenicol were added at respective concentrations of 50 µg/ml, 50 µg/ml, and 15 µg/ml and N-acetyl-D-glucosamine (GlcNAc) was added at a concentration of 100 mg/ml. The bacteria in the broth were roughly estimated using a hemocytometer.

### 1-2. Salmonella strain lineage

Serotype *gallinarum* variants were originated from the clinical isolate SG 4021 from the livers of chickens with fowl typhoid, bred in a Korean farm (Table 1), and the SG4021 strain was deposited with the Korean Research Institute of Bioscience and Biotechnology (microorganism accession number: KCTC13985BP) on October 8, 2019.

**[TABLE 1]**

| Strain | Lineage | Description |
|---|---|---|
| *S. gallinarum* | SG4021 | wild-type clinical isolate |
| | SG4022 | *relA::kan, spoT::cat* |
| | SG4023 | Δ*relA,* Δ*spoT* |
| | SG4030 | Δ*relA, ΔspoT, glmS::kan* |
| | SG4031 | Δ*relA, ΔspoT, ΔglmS* |
| | SG4032 | Δ*relA, ΔspoT*/ *GlmS⁺pLux* |
| | SG4033 | Δ*relA, ΔspoT,* Δ*glmS*/ *GlmS⁺pLux* |
| *S. typhimurium* | SHJ2037 | *relA::kan, spoT::cat* |
| | SMR2130 | Δ*relA,* Δ*spoT* |

All the Salmonella strain lineages were constructed according to the method developed by Datsenko and Wanner (Proceedings of the National Academy of Sciences 97.12 (2000): 6640-6645.), and the attenuation of Salmonella strains was induced by ppGpp deletion (ΔppGpp). The ppGpp deleted mutation was made by sequentially introducing relA::kan and spoT::cat into the genome of SG4021. In the glmS open reading frame, the gene carrying kan or cat was produced by PCR amplification using a pair of 60-nt primers, each including a 40-nt homology extension and a 20-nt priming sequence, with pKD13 serving as a template (Table 2).

**[TABLE 2]**

| Primer | | Primer base sequence (5'→3') | SEQ ID NO: |
|---|---|---|---|
| *relA::k an* | forward | GTGGATCGCAAGCCTGGGAATTTCCAGCCAGCA GTCGTGTGAGCGCTTAGGTGTAGGCTGGAGCTG CTTC | SEQ ID NO: 1 |
| | reverse | GTGCAGTCGCCGTGCATCAATCACATCCGGCAC CTGGTTCAGCTTACCGAATTCCGGGATCCGTC GACC | SEQ ID NO: 2 |
| *spoT::c at* | forward | TTAAGCGTCTTCGGCAGGCGTATCTCGTTGCAC GTGACGCTCACGAGGGCTGTAGGCTGGAGCTGC TTC | SEQ ID NO: 3 |
| | reverse | GCCAGATGTACGCGATCGCGTGCGGTAAGGCGA ATAAAGGTACTATAGACCATATGAATATCCTCC | SEQ ID NO: 4 |
| | | TTAG | |
| *glmS::k an* | forward | TTACTCAACCGTAACCGATTTTGCCAGGTTACG CGGCTGGTCAACGTCGGTGCCTTGATTGTGTAG GCTGGAGCTGCTTCGAA | SEQ ID NO: 5 |
| | reverse | ATGTGTGGAATTGTTGGCGCGATCGCGCTTCGT GATGTAGCTGAATCCTTCTTGAAGGTCATATGA ATATCCTCCTTCGTTCC | SEQ ID NO: 6 |

Then, the purified PCR amplicons were transformed by electroporation into a Salmonella strain containing a lambda Red helper plasmid (pKD46). The mutants were confirmed by PCR using original and general test primers. Before induction of SG4023 from SG4022, the antibiotic resistant gene was deleted using a helper plasmid carrying pCP20, which is a FLP recombinant enzyme.

In addition, glmS deleted mutants were produced by the method developed by Datsenko and Wanner (Proceedings of the National Academy of Sciences 97.12 (2000): 6640-6645.) (Kaiser et al., Microbiology, 146 Pt 12: 3217-3226, 2000). Salmonella cells are prone to releasing a plasmid carrying a reporter gene unnecessary for survival particularly in animals, but it is difficult to employ an antibiotic resistant gene for a selection determinant. Thus, use was made of glmS deleted mutant phenotypes that are lysed in the animal system due to the insufficiency of the peptidoglycan constituent D-glucosamine (GlcN) or N-acetyl-Dglucosamine (GlcNAc) which is an essential nutrient for. The red recombinase system of bacteriophage lambda was used to inactivate the genomic glmS gene in the Salmonella gallinarum strain.

### 1-3. GlmS+pLux plasmid

To assay tumor targeting capability through bioluminescence and compensate for glmS deleted mutation on the chromosome, the balanced-lethal host-vector system having the glmS gene of Salmonella gallinarum incorporated thereinto was constructed.

Briefly, pLux carrying the lux operon (luxCDABE, ca. 9.5kb) of Photobacterium leiognathid was inserted into the XbaI restriction enzyme site of a pUC19 plasmid backbone. For use in constructing a plasmid carrying both a lux operon cassette and glmS, the glmS gene of Salmonella gallinarum was amplified using the primers of Table 3.

**[TABLE 3]**

| Primer | | Primer base sequence (5'→3') | SEQ ID NO: |
|---|---|---|---|
| *glmS* | forward | AAGTCGACATGTGTGGAATTGTTGGC | SEQ ID NO: 7 |
| | reverse | GGGTCGACTTACTCTACGGTAACCGATTTC | SEQ ID NO: 8 |

The amplified 1.8 kb fragment was digested with Sal I and ligated to the same site of a pLux vector to construct GlmS+pLux. The glmS deleted mutant Salmonella strain transformed with the GlmS+pLux plasmid construct survives even an environment in D-glucosamine (GlcN) and N-acetyl-Dglucosamine (GlcNAc) and exhibits bioluminescence to allow for analysis through optical bioluminescent imaging.

### 1-4. Tumor cell line

From the American Type Culture Collection, murine CT26 colon carcinoma cells, 4T1 breast carcinoma cells, B16F10 melanoma cells, and human ASPC1 pancreas adenocarcinoma cells were purchased. CT26 and 4T1 cells were grown in high-glucose DMEM (Dulbecco's modified Eagle's medium) supplemented with 10% fetal bovine serum (FBS) and 1% penicillin-streptomycin. Murine B16F10 melanoma cells and ASPC1 cells were grown in RPMI 1640 supplemented with 10% FBS and 1% penicillin-streptomycin.

### 1-5. Assay for plasmid stability

Passage (1/1000) was made overnight in a flesh LB medium which was supplemented every 12 hours. Samples were taken every 24 hours and diluted. A suitable volume was spread three times over GlcNAc-supplemented LB plates irrespective of the presence or absence of ampicillin. Using the number of colonies thus formed, fractions of Salmonella spp. having the plasmid in total viable cells (colony forming unit, CFU) were calculated.

### 1-6. Mouse model construction

The cultured, various tumorous cells were xeno-grafted into the right thigh of each mouse to construct mouse tumor models. From Samtako Company (Korea), male mice at 5 to 8 weeks of age, each weighing 20 to 30 g, were purchased. Management, experiments, and euthanasia were performed on all the animals according to an approved protocol.

Mice with subcutaneous tumor were constructed as follows: the in vitro cultured tumor cells of Example 1-4 were harvested and suspended in 30 µl of PBS, followed by subcutaneous injection of 1×10⁶ cells of each of CT26, 4T1, and B16F10, and 1×10⁷ cells of ASPC1 into right thighs of the mice. For hetero-implantation of tumor cells, injection was made of CT26 and 4T1 cells into BALB/c mice, B16F10 cells into C57/BL6 mice, AsPC-1 cells into BALB/c athymic nu⁻/nu⁻ mice.

When the tumor size reached 0.5 cm³ after implantation of tumor cells (about 14 days after implantation), the ppGpp-deleted *Salmonella gallinarum* strain with mutation *glmS* of Example 1-2 including *GlmS⁺pLux* of Example 1-3 or *Salmonella typhimurium* were administered at a dose of about 1×10⁷ or about 1×10⁸ CFU/ml of PBS via tail vein (0 day, 0 dpi).

### 1-7. Assay for distribution of Salmonella spp. in mouse internal organ

To evaluate the number of live Salmonella spp., ΔppGpp *Salmonella typhimurium* and ΔppGpp *Salmonella gallinarum* strain were intravenously at ca. 1×10⁷ CFU and at ca. 1×10⁸ CFU, respectively, into mouse groups (n = 5). Days 1, 5, 10, and 16 after injection, the mice were sacrificed. Organs were excised and homogenized by a homogenizer in sterile PBS containing 0.05% Tween-20. Salmonella spp. were recovered from the homogenate and plated onto agar plates containing 50 µg/ml kanamycin and 15 µg/ml chloramphenicol.

### 1-8. Analysis for tumor size and mouse survival rate

Tumor volumes were calculated according to the following formula.

Tumor volume (mm³) = (tumor length × tumor height × tumor width)/2

All animal experiments were approved by the Chonnam National University Institutional Animal Care and Use Committee (NO. CNU IACUC-H-2016-15). According to the instruction, mice with a tumor volume of 1,500 mm³ or larger were sacrificed, and the survival rates of mice were evaluated according to the Gehan-Breslow-Wilcoxon test.

### 1-9. Optical bioluminescence imaging

In order to image the bioluminescence of Salmonella spp. as an index for targeting tumors, mice were anesthetized with 2% isoflurane and then placed in a light-blocked chamber of CCD (charged couple detector) camera-installed IVIS100 (Caliper, Hopkinton, MA, USA). The photons emitted from luciferase-expressing Salmonella spp. were collected and integrated for 1 minute. Pseudo color images representing counted photons were overlaid on bright optical images of rats using living image software version v.2.25 (Xenogen-Caliper, Hopkinto, Mass.).

### 1-10. Immunofluorescent staining and confocal microscopy

In order to stain Salmonella strains in tumors, the isolated tissues were fixed overnight at room temperature with 3.4 % paraformaldehyde in PBS and embedded into Optimal Cutting Temperature compound (OCT; Tissue-Tek). Subsequently, the tissues were frozen and sectioned into 7-mm thick slices using a microtome-cryostat. The tissue slices were collected on aminopropyl triethoxysilane-coated slides. The slides were washed with PBS (pH 7.4) to completely remove OCT and incubated overnight at 4°C with an anti-Salmonella LPS antibody (1 : 100, Abcam # ab8274). Thereafter, Alexa Fluor 568-conjugated goat anti-mouse (1 : 100, Life Technologies A11031) antibody and Alexa Fluor 488-conjugated Phallodin (1 : 1000, Invitrogen # W21404) were used as secondary antibodies to detect F-actin. After the nuclei were stained with DAPI/Antifade (1 : 200, Invitrogen), the samples were mounted. Stained images were captured using Zeiss confocal microscope LSM 510 (Zeiss Laboratories), and representative images are given unless otherwise stated.

### 1-11. Statistical Analysis

Statistical analyses were performed using SPSS 18.0 statistical package (SPSS Inc., Chicago, IL, USA). Statistical significance of tumor growth between control and test groups was determined using two-tailed Student's t-test. P values < 0.05 were considered statistically significant, and all data are expressed as mean ± SD.

### EXAMPLE 2. Assay for In vivo Toxicity of Salmonella gallinarum strains and for Plasmid Stability

### 2-1. Assay for in vitro toxicity of Salmonella gallinarum strains

In order to apply *Salmonella gallinarum* strains to bacterial cancer therapy, wild-type *Salmonella gallinarum* was injected intravenously (i.v.) into the mice which were then analyzed for survival.

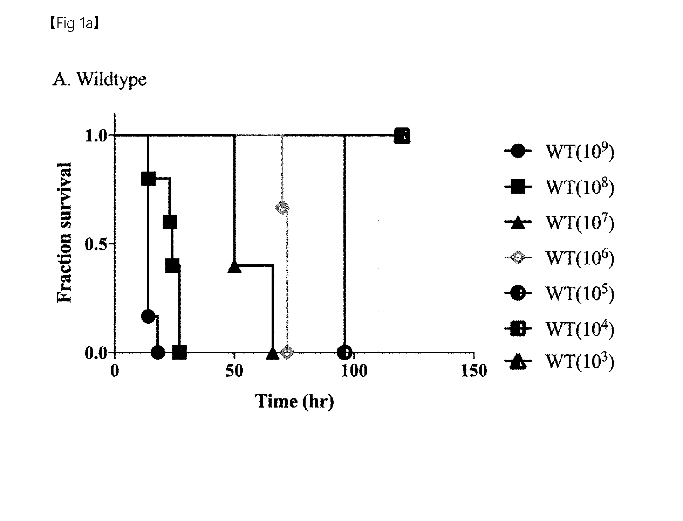

As a result, even though causing typhoid only in fowls, the intravenous injection of the wild-type *Salmonella gallinarum* strain (SG4021) induced death of all mice at a dose of about 10⁵ CFU or greater (FIG. 1A).

### 2-2. Determination of dosage according to in vivo toxicity of attenuated Salmonella gallinarum strain

Since the wild-type *Salmonella gallinarum* strain was observed to exhibit in vivo toxicity in Example 2-1, *Salmonella gallinarum* strain was attenuated by blocking the synthesis of ppGpp.

Briefly, the ppGpp-deleted (ΔppGpp) mutant *Salmonella gallinarum* strains of Example 1-2 in which both ppGpp synthetase I and II respectively encoded by *relA* and *spoT* genes had been mutated, and the *Salmonella typhimurium* were intravenously injected to BALB/c mice which were then analyzed for survival rate.

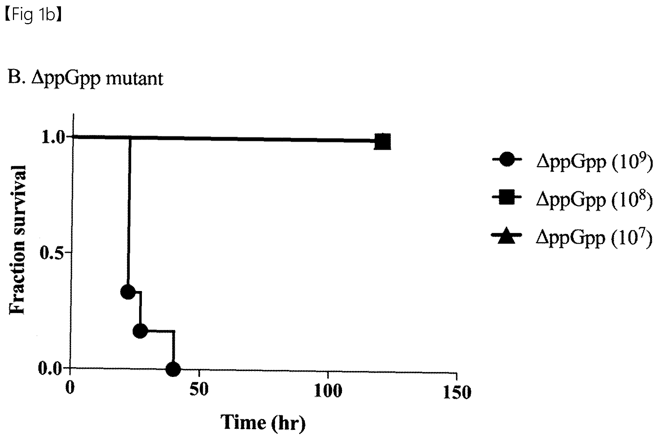

As a result, the mice were observed to survive up to10⁸ CFU of the ΔppGpp *Salmonella gallinarum* strain (SG4023) (FIG. 1B) and up to about 10⁷ CFU of the ΔppGpp Salmonella typhimurium strain. Meanwhile, similar experiments were carried out with Salmonella typhimurium A1-R that can selectively infect and attack living tumors. As a result, it was observed that strains with modified lipopolysaccharide, msbB-mutation, (VN20009), and Δ*rfaG*/Δ*rfaD* double mutation were less prone to induce an immune response in the range of about 10⁶ to about 10⁷ CFU. Mice with tumors were observed to survive the intravenous infection of about 10⁸ CFU of *E. coli.*

It was understood from the data that with the assumption that E. coli was almost free of toxicity, the maximal dose of Salmonella spp. that animals free of nonspecific catastrophe can endure was about 10⁸ CFU.

### 2-3. Assay for plasmid stability

With respect to the stability of the transformed plasmids, the same procedure as in Example 1-5 was carried out to evaluate whether the plasmids were lost.

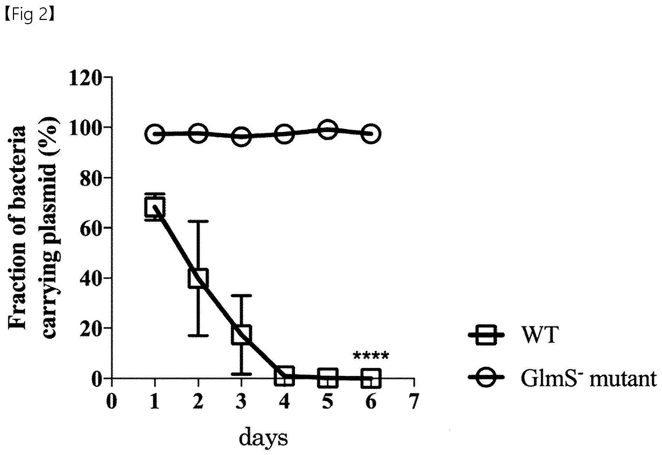

As a result, 99% of the plasmids carried by the wild-type *Salmonella gallinarum* strain (SG4021) were lost by day 4 while the plasmids carried by the *glmS-*deleted *Salmonella gallinarum* strain (SG4031) were completely maintained (FIG. 2).

Subsequent experiments employed the ΔppGpp strain, transformed with *GlmS⁺pLux* of Example 1-3, having a mutant *glmS* gene on the genome thereof so as to visualize *Salmonella gallinarum* strains in mice.

### EXAMPLE 3. Assay for Tumor Targeting of ΔppGpp Salmonella gallinarum Strain

### 3-1. Examination of tumor targeting according to bioluminescence signal imaging

To examine the tumor targeting of the ΔppGpp *Salmonella gallinarum* strain, bioluminescence signals of the *Salmonella gallinarum* strain were imaged in the same manner as in Example 1-9.

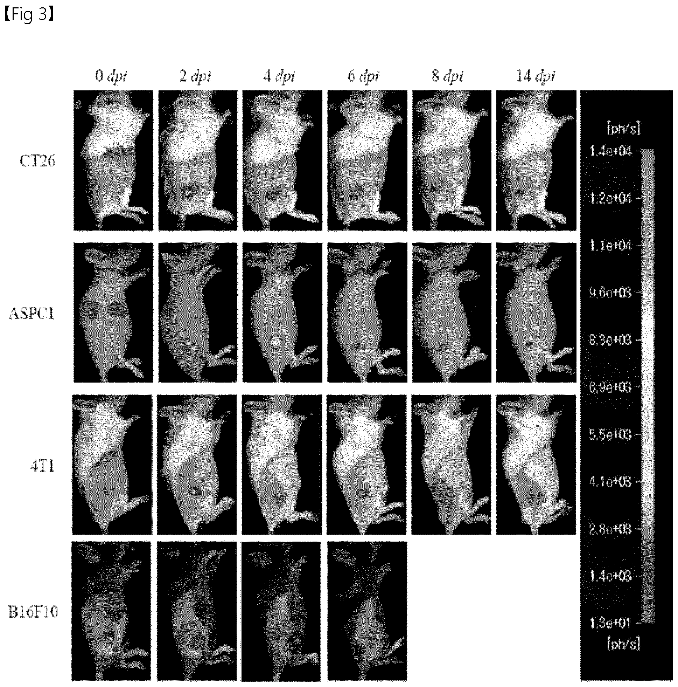

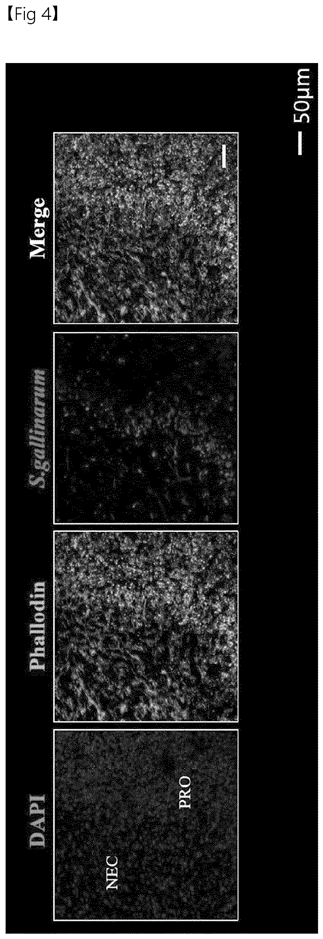

As a result, bioluminescence signals were detected mainly from internal organs (liver and spleen) immediately after injection of the ΔppGpp *Salmonella gallinarum* strain (SG4031) (20 min, 0 day post injection (dpi)). Bioluminescence signals appeared only in the implanted tumor tissues. After appearance of peaks for AsPC-1 at 4 dpi and other tumors at 2 dpi, the bioluminescence signals were gradually diminished. Particularly, the *Salmonella gallinarum* strain was observed to target all the tumors implanted in three different mouse lines (FIG. 3). The presence of the ΔppGpp *Salmonella gallinarum* strain in implanted tumor tissues was confirmed at 3 dpi by staining the Salmonella strain in the implanted CT26 cells, with the detection of the Salmonella strain at the interface between a proliferative region and a necrotic region (FIG. 4).

### 3-2. Retention of plasmid in mouse

The wild-type and the mutant ΔppGpp *Salmonella gallinarum* strain of Example 1-2 including the *GlmS⁺pLux* carrying the mutant *glmS* gene of Example 1-3 were injected into Balb/c mice including CT26 cells and then imaged at 0, 2, and 4 dpi according to the method of Example 1-9.

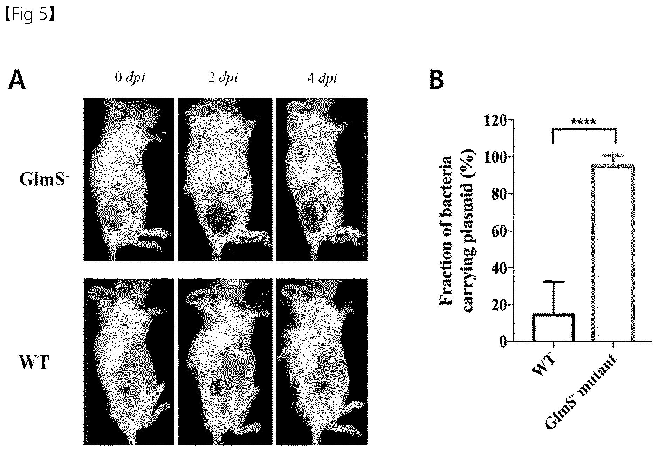

As a result, a strong bioluminescence signal was retained in the glmS-deleted ΔppGpp *Salmonella gallinarum* strain (SG4031), but not in the ΔppGpp *Salmonella gallinarum* strain (SG4023) (FIG. 5A). In addition, when the tumor-related *Salmonella gallinarum* strains were calculated at 7 dpi on ampicillin-containing plates, more than 90% of the wild type was found to lose the plasmids (FIG. 5B), demonstrating the excellency of the balanced-lethal host-vector system.

### EXAMPLE 4. Anticancer Effect of ΔppGpp Salmonella gallinarum Strain on CT26 Tumor Mouse Model

### 4-1. Prolonged duration of life of Salmonella gallinarum-treated mouse

To CT26 cell-implanted BALB/c mice, ΔppGpp *Salmonella gallinarum* was intravenously injected at a dose of about 1×10⁸ CFU while the ΔppGpp Salmonella typhimurium was used as a control (ca. 1×10⁷ CFU). In vivo anticancer activity of the ΔppGpp *Salmonella gallinarum* strains was analyzed according to Examples 1-8 and 1-10.

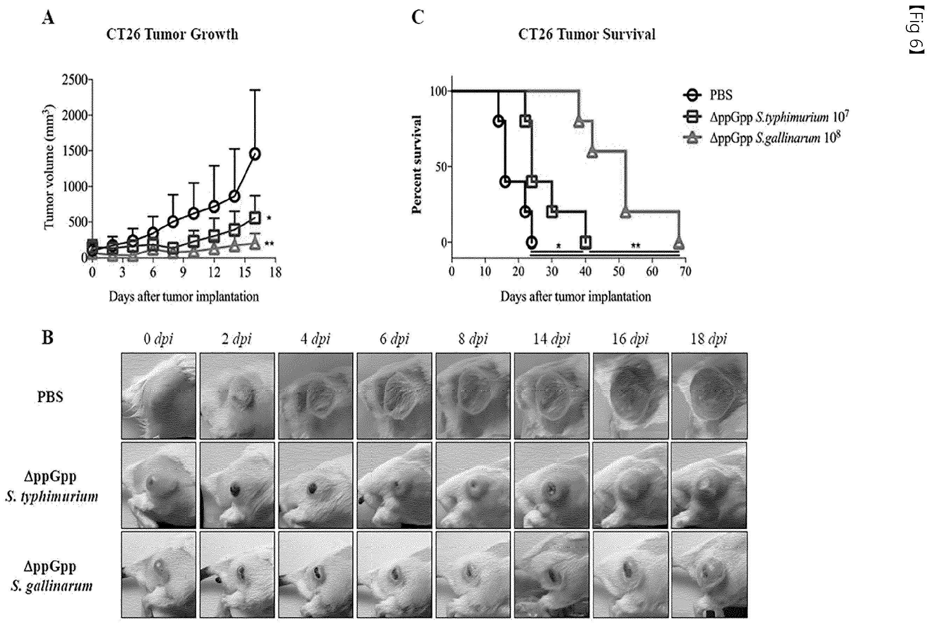

Treatment of about 1×10⁸ CFU of the ΔppGpp *Salmonella gallinarum* strain (SG4023) was observed to significantly improve the inhibitory effect of tumor growth in the mice, compared to the PBS control and about 1×10⁷ CFU of the Salmonella typhimurium strain (SMR2130) (FIGS. 6A and 6B). That is, the group treated with the ΔppGpp *Salmonella gallinarum* group exhibited a prolonged duration of life, compared to the group treated with the ΔppGpp Salmonella typhimurium strain or PBS (FIG. 6C). The mice treated with about 1×10⁷ CFU of the Salmonella typhimurium strain exhibited an average duration of life of about 24 days while the mice treated with about 1×10⁸ CFU of *Salmonella gallinarum* survived for 53 days on average. Furthermore, the duration of life of the mice treated with 1×10⁸ CFU of the *Salmonella gallinarum* remarkably increased (3.5 folds), compared to the PBS control (15 days).

### 4-2. Distribution pattern of Salmonella gallinarum strain in mouse

To examine distribution patterns of *Salmonella gallinarum* strains in mice, temporal changes in the number of the *Salmonella gallinarum* strains were monitored in mouse organs.

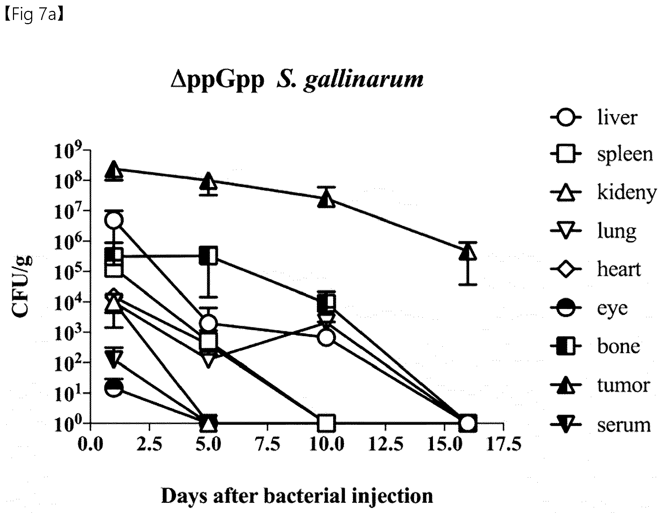

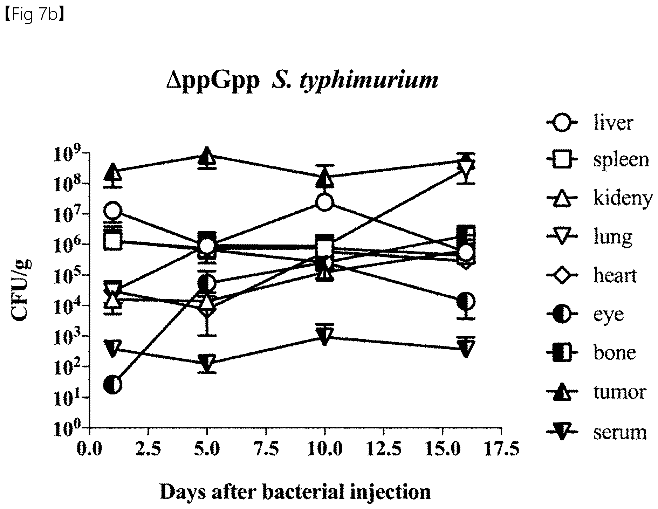

As a result, maximal highest levels (10⁸ to 10⁹ CFU/g) in tumors were observed in both of the Salmonella strains one day after the injection (1dpi). The ΔppGpp Salmonella typhimurium strain (SHJ2037) was maintained at the same level in tumor while the number of the ΔppGpp *Salmonella gallinarum* strain (SG4022) was gradually reduced into 10⁶ CFU/g over 16 days. At 1 dpi, the Salmonella typhimurium strain was maintained at a level of 10⁶ to 10⁷ CFU/g in liver, spleen, and bone, at a level of 10⁶ to 10⁷ CFU/g, at a level of 10⁴ CFU/g in lung, kidney, and heart, and at a level of 10¹ and 10³ CFU/g in serum and eye, and the levels were not significantly reduced over 16 days. In contrast, the *Salmonella gallinarum* strain was found to exhibit similar distribution patterns for the organs at 1 dpi. The *Salmonella gallinarum* strain was completely cleared from the organs by 16 dpi (FIG. 7).

From the data obtained above, it is understood that the ΔppGpp *Salmonella gallinarum* strain possessing the mutant *glmS* gene on the chromosome thereof specifically targets tumors to exhibit an anti-tumor effect and as such, can be used for anti-tumor purposes.

The disclosure has been described above with reference to the preferred embodiments. It will be understood by those skilled in the art that the disclosure may be embodied in various other forms without departing from the spirit or essential characteristics thereof. Therefore, the above-described embodiments should be considered in an illustrative rather than a restrictive sense. The scope of the disclosure is defined by the appended claims rather than by the foregoing description, and all differences within the scope of equivalents thereof should be construed as being included in the disclosure.

## Claims

1. An attenuated *Salmonella gallinarum* strain, lacking guanosine tetraphosphate (ppGpp) production capability.

2. The attenuated *Salmonella gallinarum* strain of claim 1, wherein the strain is derived by deleting guanosine tetraphosphate production capability from *Salmonella gallinarum* SG4021 (microorganism accession number: KCTC13985BP).

3. The attenuated *Salmonella gallinarum* strain of claim 1, wherein the strain is *Salmonella gallinarum* SG4023 (microorganism accession number: KCTC14338BP).

4. The attenuated *Salmonella gallinarum* strain of claim 1, wherein the strain lacks a gene coding for a ppGpp synthetase.

5. The attenuated *Salmonella gallinarum* strain of claim 4, wherein the gene includes *relA* and *spoT.*

6. The attenuated *Salmonella gallinarum* strain of claim 1, wherein the strain lacks a *glmS* gene.

7. The attenuated *Salmonella gallinarum* strain of claim 6, wherein the strain is *Salmonella gallinarum* SG4031 (microorganism accession number: KCTC14339BP).

8. The attenuated *Salmonella gallinarum* strain of claim 6, wherein the strain is transformed with a plasmid comprising the *glmS* gene operatively linked to a promoter.

9. The attenuated *Salmonella gallinarum* strain of claim 8, wherein the plasmid further comprises a luminescence gene.

10. The attenuated *Salmonella gallinarum* strain of claim 9, wherein the luminescence gene is *luxCDABE.*

11. A pharmaceutical composition for prevention or treatment of a tumor, the composition comprising the strain of anyone of claims 1 to 10.

12. The pharmaceutical composition of claim 11, wherein the tumor is a solid cancer.

13. The pharmaceutical composition of claim 11, wherein the tumor is adenocarcinoma.

14. The pharmaceutical composition of claim 11, wherein the tumor is a cancer selected from the group consisting of colorectal cancer, pancreatic cancer, skin cancer, and breast cancer.

15. A method for providing information about tumor analysis, the method comprising the steps of:
administering the *Salmonella gallinarum* strain to a subject;
detecting bioluminescence of the strain; and
determining the presence of tumor in the subject if the bioluminescence is detected.


<110>     ChoyBiolab Co., Ltd.
          INDUSTRY FOUNDATION OF CHONNAM NATIONAL UNIVERSITY

<120>     Tumor Targeting Salmonella gallinarum and Use Thereof

<130>     PN190380

<150>     KR 10-2019-0138224
<151>     2019-10-31

<160>     8

<170>     KoPatentIn 3.0

<210>     1
<211>     70
<212>     DNA
<213>     Artificial Sequence

<220>
<223>     relA::kan_forward


<400>     1
gtggatcgca agcctgggaa tttccagcca gcagtcgtgt gagcgcttag gtgtaggctg          60

gagctgcttc                                                                  70


<210>     2
<211>     70
<212>     DNA
<213>     Artificial Sequence

<220>
<223>     relA::kan_reverse


<400>     2
gtgcagtcgc cgtgcatcaa tcacatccgg cacctggttc agcttaccga attccgggga          60

tccgtcgacc                                                                  70


<210>     3
<211>     69
<212>     DNA
<213>     Artificial Sequence

<220>
<223>     spoT::cat_forward


<400>     3
ttaagcgtct tcggcaggcg tatctcgttg cacgtgacgc tcacgagggc tgtaggctgg          60

agctgcttc                                                                   69


<210>     4
<211>     70
<212>     DNA
<213>     Artificial Sequence

<220>
<223>    spoT::cat_reverse


<400>    4
gccagatgta cgcgatcgcg tgcggtaagg cgaataaagg tactatagac catatgaata        60

tcctccttag                                                               70


<210>    5
<211>    83
<212>    DNA
<213>    Artificial Sequence

<220>
<223>    glmS::kan_forward


<400>    5
ttactcaacc gtaaccgatt ttgccaggtt acgcggctgg tcaacgtcgg tgccttgatt        60

gtgtaggctg gagctgcttc gaa                                                83


<210>    6
<211>    83
<212>    DNA
<213>    Artificial Sequence

<220>
<223>    glmS::kan_reverse


<400>    6
atgtgtggaa ttgttggcgc gatcgcgctt cgtgatgtag ctgaatcctt cttgaaggtc        60

atatgaatat cctccttcgt tcc                                                83


<210>    7
<211>    26
<212>    DNA
<213>    Artificial Sequence

<220>
<223>    glmS_forward


<400>    7
aagtcgacat gtgtggaatt gttggc                                             26


<210>    8
<211>    30
<212>    DNA
<213>    Artificial Sequence

<220>
<223>    glmS_reverse

<400>    8
gggtcgactt actctacggt aaccgatttc                                        30